# EUROPEAN PATENT APPLICATION

(11) **EP 2 289 529 A1**
(43) Date of publication of application: **02.03.2011**
(21) Application number: 09166018.3
(22) Date of filing: 21.07.2009
(51) Int. Cl.: A61K 36/71, A23K 1/00, A23L 1/39, A23L 2/02, A61K 9/48, A61P 25/00

(54) **Nigella extracts for the treatment of disorders connected to impaired or imbalanced neurotransmission**

(71) Applicant: DSM IP Assets B.V., 6411 TE Heerlen (NL)
(72) Inventor: The designation of the inventor has not yet been filed
(74) Representative: Mezger, Wolfgang

(57) **Abstract**

The present invention relates to the use of *Nigella sp.* extracts and their volatile components for the treatment of disorders connected to impaired neurotransmission in animals including humans as well as to dietary supplements, food and feed or pharmaceutical compositions containing such extracts or their volatile components.

## Description

### FIELD OF THE INVENTION

The present invention relates to the use of lipophilic *Nigella sp.* seed extracts as agents for the treatment of disorders connected to impaired, i.e. reduced neurotransmission. In addition, it also relates to dietary and pharmaceutical compositions containing such *Nigella sp.* seed extracts.

### BACKGROUND OF THE INVENTION

*Nigella sativa* is commonly called fennel flower, nutmeg flower, Roman coriander, blackseed, black carroway, black onion, or black cumin (although *Bunium persicum* is also referred to as black cumin).

Traditionally, *Nigella sativa* has been thought of as having anti-hypertensive properties, carminative properties (i.e. preventing/removing gas in the gastrointestinal tract) or anti-helminic properties. Total seed extracts have also been traditionally used to stimulate the body's energy and help recovery from fatigue and dispiritedness.

### DETAILED DESCRIPTION OF THE INVENTION

It has been surprisingly found that lipophilic extracts of *Nigella sp.,* especially *Nigella sativa* seeds are serotonin re-uptake inhibitors, thus prolonging the time that serotonin is available for neurotransmission. Thus, they can mimic the action of the so-called selective serotonin re-uptake inhibitors "SSRIs" such as PROZAC, and can thus promote a balanced mood and relieve stress. Thus, they are particularly useful for treating depression, anxiety, or a combination thereof, mediated by misbalanced serotonin neurotransmission or a misbalanced serotonin neurotransmission in combination with noradrenalin and dopamine neurotransmission. The extracts also useful in the treatment of other diseases, such as genitourinary diseases (for treatment of stress and urge incontinence) and neurological pain associated with depression, or other conditions which have been classically treated by pharmaceuticals which are SSRIs.

This invention also relates to the use of lipophilic *Nigella sp.,* especially *Nigella sativa* seed extracts for the manufacture of compositions for the treatment and prevention of disorders connected to impaired or reduced neurotransmission. Thus, in one aspect the present invention relates to dietary or pharmaceutical, or veterinary compositions comprising at least one lipophilic *Nigella sp.,* especially *Nigella sativa* seed extract for the treatment of disorders connected to impaired or reduced neurotransmission.

### BRIEF DESCRIPTION OF THE FIGURES

FIGURE 1 shows the measured IC₅₀ values for inhibition of serotonin uptake by *Nigella sativa* lipophilic extract is 14.14 ± 4.5 µg/mL.
FIGURE 2 shows the IC₅₀ values for inhibition of serotonin uptake by a cold-pressed *Nigella sativa* extract. As can be seen, cold-pressed *Nigella sativa* extract does not inhibit serotonin uptake.
FIGURE 3 shows a chromatogram of a *Nigella sativa* extract containing fatty acids and 0.01 % (w/w) thymoquinone.

The main neurotransmitters are serotonin, dopamine, noradrenaline, acetylcholine, glutamate, gamma-amino-butyric acid. Those neurotransmitters of particular relevance to mood-related disorders are serotonin, noradrenaline, and dopamine. Increase in neurotransmission is achieved by increasing the concentration of the neurotransmitter in the synaptic cleft thus making it available for increased or prolonged neurotransmission through inhibition of re-uptake into the pre-synaptic nerve end, or by preventing neurotransmitter catabolism by inhibition of degrading enzymes such as monoamine oxidase A and B.

### DEFINITIONS

The terms "impaired neurotransmission" and "reduced neurotransmission" are used interchangeably throughout the present application. They are used in the present application in accordance with their meaning well-known to the person skilled in the art, and relate to a state of deregulation of neurotransmission, which may occur at the level of neurotransmitter biosynthesis, processing, storage, release, re-uptake and receptor binding. Impaired neurotransmission, in particular a reduction of neurotransmission, may manifest itself in animals including humans as a disturbance of behavior, emotions, mood and thinking processes, for example, in one of various types of depression.
The term "lipophilic seed extract" means that the seeds have been extracted using a lipophilic organic solvent which is suitable (i.e. approved by regulatory agencies) for food use, or alternatively, a water-based distillation which yields essential oils. Examples of suitable organic solvents include: liquid carbon dioxide under supercritical conditions, ethanol, ethyl acetate, methyl-tert.-butylether/Methanol, propane, butane, acetone and nitrous oxide. These extracts can be made using known techniques, and typically contain a variety of potentially active ingredients, such as: nonterpenoid hydrocarbons, monoterpenoid hydrocarbons, monoterpenoid ketones, monoterpenoid alcohols, sesquiterpenoid hydrocarbons and phenyl propanoid compounds. "Lipophilic seed extract" as used herein, specifically excludes extracts which contain only the so-called fixed oil fraction, which are obtained by cold-pressed methods and which contain only fatty acid components.
*"Nigella sp.*" may include any member of the *Nigella* species. A preferred plant is *Nigella sativa*, but other species of *Nigella* may also be used, such as *N. damascena L.*
"Animals" includes humans, and encompasses mammals, fish and birds. Preferred are: humans, pets or companion animals, farm animals, and animals used in the fur industry.
"Farm animals" includes: fish, such as salmon and trout, aquaculture animals such as shrimp, pigs, horses, ruminants (cattle, sheep, goats) and poultry (such as geese, chickens, broilers, laying hens, quails, ducks, and turkeys). Preferred are poultry, cattle, sheep, goats and pigs.
"Pets" or "companion animals" include dogs, cats, birds, aquarium fish, guinea pigs, (jack) rabbits, hares and ferrets. Dogs and cats are preferred.
"Animals used in the fur industry" include minks, foxes, and hares.
"Dietary compositions" includes any type of nutritional product, such as (fortified) food/feed and beverages, and also includes clinical nutrition products, and dietary supplements.
"Fortification" means that a *Nigella sp.,* preferably a *Nigella sativa* extract was added during manufacture of the food/feed or beverage.
"Prevent" refers to prophylactic treatments as well as to delayed onset of symptoms, reduction in symptoms if they appear, early invtervention, and generally lessening the risk of symptoms or a conditions.

In humans, disorders connected to impaired or reduced neurotransmission, and thus those disorders which are treated or prevented by the medicaments of this invention include: depression, anxiety, irritability, unhappiness/discontentedness, sadness, dysphoria, dysthymia, obsessive-compulsive behaviors, insomnia, and biorhythm abnormalities (disturbed circadian rhythms).

The medicaments of this invention can thus be characterized as mood balancing agents, mood/vitality improvers, stress relievers, anxiety reducers, tension reducers, relaxants, sleep improvers, and normalizers of biorhythms.

Numerous pharmaceutical compositions which are neurotransmitter regulators have proven helpful in various mood-related disorders. As the extracts of this invention have been found to work using the same or similar biochemical pathways, then it can be concluded that they are useful for similar conditions as uptake inhibitors.

Compounds that increase neurotransmitter levels in the brain and thus enhance their transmission, can therefore exhibit antidepressant properties as well as beneficial effects on a variety of other mental disorders (Neurotransmitters, Drugs and Brain function, R.A. Webster (ed), John Wiley & Sons, New York, 2001, p. 187-211, 289-452, 477-498).

Medicaments for the treatment of disorders connected to impaired or imbalanced neurotransmission encompass antidepressants, mood improvers, stress relievers, condition improvers, anxiety reducers and obsessive-compulsive behaviour reducers. They all improve, enhance and support the physiological neurotransmission, especially in the central nervous system, and therefore alleviate mental malfunction.

Tricyclic antidepressant compounds (TCAs) such as imipramine, amitriptyline, and clomipramine, inhibit the re-uptake of serotonin, noradrenaline and/or dopamine. They are widely regarded as among the most effective antidepressants available, but they have a number of disadvantages because they additionally interact with a number of brain receptors, e.g., with cholinergic receptors. Most importantly, TCAs are risky because, taken in overdose, they frequently show acute cardiotoxicity.

Another class of antidepressant drugs are the so-called SSRIs (selective serotonin re-uptake inhibitors), including fluoxetine, paroxetine, sertraline, citalopram, and fluvoxamine, that block the serotonin transporter (SERT), a high affinity sodium chloride-dependent neurotransmitter transporter that terminates serotonergic neurotransmission by reuptake of serotonin. They have been proven to be effective in the treatment of depression and anxiety, and are usually better tolerated than TCAs. These medications are typically started at low dosages and may be increased until they reach a therapeutic level. A common side effect is nausea. Other possible side effects include decreased appetite, dry mouth, sweating, infection, constipation, yawning, tremors, sleepiness and sexual dysfunction.

Also, systematic reviews and placebo-controlled randomized clinical trials (RCTs) indicate that some SSRIs (escitalopram, paroxetine and sertraline), the SNRI venlafaxine, some benzodiazepines (alprazolam and diazepam), the tricyclic antidepressant imipramine, and the 5-HT_{1A} partial agonist buspirone are all efficacious in acute treatment. In general, the effect of treatment is often moderate and symptoms reappear when the treatment period is discontinued. Therefore, a continuous long-term treatment or prevention with compounds which have fewer side effects than SSRIs and can be taken over long time periods might be favorable over drug treatment. This can be achieved by supplementing persons at need with the *Nigella sp.,* and preferably the *Nagella sativa* extracts of this invention in the form of dietary supplements.

Therefore, there is still a need for compounds for the treatment of disorders connected to impaired neurotransmission which do not show the negative side effects of known antidepressants. Many patients are interested in alternative therapies with fewer or no side effects associated with the taking of known drugs. Severe depression is a long-lasting and recurring disease, which is usually poorly diagnosed. Furthermore, many patients suffer from mild or moderately severe depression. Thus, there is an increasing interest in the development of compounds, as well as of pharmaceutical and/or dietary compositions, that can be used to treat or prevent the development of mental diseases/disorders such as depression, in people at risk, and to stabilize mood.

Furthermore, modulators of neurotransmission exert pleiotropic effects on mental and cognitive functions. In addition to depression, examples of other human conditions are listed below.

### General Anxiety Disorder (GAD) and other Anxiety Disorder

It is well known that impaired neurotransmission, e.g. low neurotransmitter levels, is connected to various mental diseases and conditions such as depression and generalized anxiety disorders (GAD). Patients often suffer, either as a comorbidity to depression or alone, from generalized anxiety syndrome or generalized anxiety disorder. GAD is a highly prevalent anxiety condition and chronic illness in primary care (∼10% of patients) (Wittchen et al, 2005 Eur. Neuropsychopharm. 15: 357-376). Patients present to their primary care physician with multiple physical symptoms. GAD is characterized by chronic tension, and anxious worrying and tension (>6 months), which are disabling and uncontrollable, and accompanied by a characteristic hypervigilance syndrome (including restlessness, muscle tension, and sleep problems). If untreated, GAD runs a chronic, fluctuating course and tends to become more severe with age. GAD patients suffer from sub-syndromal depression. GAD patients are classified as high utilizers with the highest overall direct and indirect health economic burden of all anxiety and depressive disorders. Despite high GAD incidence, few sufferers are diagnosed, prescribed medication, or receive psychiatric referral; simple diagnostic tools to aid patient recognition and monitoring are needed. Regardless of specific diagnosis, physicians require effective GAD-symptom treatments. SSRIs such as paroxetine, are effective for GAD treatment [Stocchi et al.,2003 *J Clin Psychiatry* 63(3):250.]

A number of SSRIs have been approved by the FDA for treatment of anxiety disorders:
- fluoxetine: - OCD, PD
- Sertraline: - OCD, PD, PTSD, SAD
- paroxetine: - OCD, PD, SAD, GAD, PTSD
- escitalopram: - GAD
- fluvoxamine: - OCD

[OCD: obsessive-compulsive disorder, PD: panic disorder, PTSD: post-traumatic stress disorder, SAD: social anxiety disorder, GAD: generalised anxiety disorder]

### Aggression

Pathological impulsive aggressivity may be associated with lower serotonergic innervation in the anterior cingulate cortex, as demonstrated using PET, where [¹¹C]McN 5652 binding was shown to be significantly reduced in this brain region [Frankle et al. 2005 Am. J. Psych, 162: 915-923]. In this respect, it also must be noted that serotonin dysfunction has been correlated with impulsive and violent criminal behaviours, alcohol abuse and suicide attempts, as well as being reported in children institutionalised for aggressive behavior.

Dietary depletion of tryptophan has been demonstrated to result in an increase in aggressive responses, in a laboratory situation, whereas augmentation of tryptophan resulted in a decrease in aggressive responses. Thus, presumably, decreased serotonin is linked to an increase in aggression [Marsh et al. 2002, Neuropsychopharm 26:660-671].

### Attention deficit hyperactivity disorder (ADHD)

A number of antidepressants which affect reuptake of one or more of the monoamines are also effective in the treatment of ADHD and are a good alternative to the commonly-used stimulant medications: (1) tricyclic antidepressants (TCAs), such as imipramine, desipramine and amitriptyline, whose mechanism of action includes the blockade of noradrenaline and serotonin reuptake and downregulation of β-adrenergic receptors; (2) dual reuptake inhibitors, such as bupropion (dopamine/noradrenaline reuptake inhibitor) and venlafaxine (SNRI); (3) single reuptake inhibitors, such as atomoxetine and tomoxetine (blockade of prefrontal cortex presynaptic noradrenaline transporters) [Greydanus 2005. Ind. J. Ped., 72:953-960; Chouinard 2005 J. Psych. & Neuro., 31(3): 168-176].

### Circadian Rhythm Disturbances

Mood disorders and occupational stress can lead to disturbances in circadian rhythms (so-called biorhythms). These conditions are often chronic and persistent. Also, deregulation of circadian rhythms induced by long-distance flights (jet-lag), as well as by shift-work, can cause similar symptoms and distress. Therefore, treatment with dietary supplementation to maintain the normal circadian rhythm (that an animal or human is used to), and/or to alleviate and prevent symptoms associated with a disturbed circadian rhythm, such as impairment of cognitive function and memory, and mental and physical fatigue, is warranted to improve the overall quality of life and to benefit the vital energy of a person in need thereof.

### Sleep Disorder, Insomnia, and Chronic Fatigue Syndrome

Sleep and depression are closely linked. Sleep EEG abnormalities are usual in depression, and insomnia can lead to depression. Sleep alterations affect other biological rhythms involved in depression (Vogel et al. 1990 Neurosci Biobehav Rev 14:49-63; Mc Carley 1982. Am J Psych. 139:565-570). Antidepressants can improve sleep continuity, reduce rapid eye movement (REM) sleep and prolong slow wave (SWS) sleep (Sraner et al 2006 in Clinical Pharmacology of Sleep. S.R. Pandi-Perumal et al (eds). Birkhäuser, Basel, , pp 103- 124.

TCAs are commonly used for treatment of chronic fatigue syndrome. TCAs are believed to promote stage 4, non-rapid eye movement sleep, [Craig, et al 2002 Am. Fam. Physician, 65:1083-1090].

### Obsessive-compulsive disorder (OCD)

Enhanced neurotransmission at 5-HT₂ receptors may be implicated in the therapeutic action of SSRIs in OCD; hyperactivity in the neuronal loop between the orbitofrontal cortex, head of the caudate nucleus and thalamus may be attenuated by SSRIs, due to increased activation of inhibitory 5-HT₂ receptors in the orbitofrontal cortex [Blier et al 2001 J. Psych & Neuro. 26(1):37-43]. SSRIs, such as zimeldine, fluoxetine and sertraline, have been demonstrated to be effective in the treatment of OCD [Chouinard, 2005 J. Psych & Neuro. 31(3):168-176].

### Pain

Antidepressants with differing mechanisms of action can also be effective in the treatment of pain. For example, amitriptyline and mianserin, which are potent 5-HT₂ antagonists, are used for the control of chronic pain [Blier, et al 2001. J. Psych & Neuro, 26(1): 37-43]. The noradrenaline/serotonin/dopamine reuptake inhibitor, duloxetine, is efficacious in the treatment of neuropathic pain associated with diabetic peripheral neuropathy [Chouinard, 2005. J. Psych & Neuro. 31(3): 168-176]. This type of pain is different from that associated with inflammation. According to this invention, pain is reduced using a different mechanism than decreasing inflammation.

Thus, the invention relates to a method for the treatment or prevention of a symptom or disorder connected to impaired neurotransmission, said method comprising administering an effective amount of a lipophilic *Nigella sp,* or preferably a *Nigella sativa* seed extract to an animal (including humans) which is in need thereof.

As an antidepressant, lipophilic *Nigella sp.,* and preferably *Nigella sativa* seed extracts as well as compositions/medicaments containing them, are thus medicaments for treating mental, behavioural and emotional/affective, neurotic, neurodegenerative, eating and stress related disorders such as e.g. unipolar depression, bipolar depression, acute depression, chronic depression, subchronic depression, dysthymia, dysphoria, postpartum depression, premenstrual dysphoria/syndrome (PMS), climacteric depressive symptoms, aggression, attention deficit disorders (ADS), social anxiety disorders, seasonal affective disorders, anxiety/generalized anxiety disorders (GAD), fibromyalgia syndrome, chronic fatigue, sleep disorders (insomnia), post-traumatic stress disorders, panic disorders, obsessive compulsive disorders, restless leg syndrome, nervousness, migraine/primary headaches and pain in general, emesis, bulimia, anorexia nervosa, binge eating disorder, gastrointestinal disorders, burn out syndrome, irritability and tiredness.

As an antidepressant, lipophilic *Nigella sp.,* and preferably *Nigella sativa* seed extracts as well as compositions/medicaments containing them, can also be used for the manufacture of compositions for primary and secondary prevention and/or the treatment of neurocognitive impairment. Furthermore they are also effective in the treatment of depressive symptoms or other symptoms related to disturbed neurotransmission occurring as comorbidity in chronic diseases, e.g. cardiovascular diseases, strokes, cancer, Alzheimer's Disease, and Parkinson's disease. In accordance with this invention the lipophilic *Nigella sp.,* preferably *Nigella sativa* seed extracts as well as compositions/medicaments containing them, are *not* used for treating cardiovascular diseases, strokes, cancer, Alzheimer's disease and Parkinson's disease themselves, but to treat depression evoked by these diseases.

The lipophilic *Nigella sp.* and preferably *Nigella sativa* seed extracts can be used for the manufacture of medicaments for the treatment of a disorder connected to impaired or imbalanced neurotransmission. They can additionally be used for the manufacture of compositions for use as mood balancing agents, mood/vitality improvers, stress relievers, condition improvers, reducers of anxiety, reducers of tension, reducers of sadness, reducers of unhappiness/discontent, reducers of irritability, reducers of dysphoria, reducers of obsessive-compulsive behaviour, relaxants, sleep improvers and/or insomnia alleviators.

"Mood improver", "vitality improver" or "emotional wellness booster" means that the mood or vitality of a person treated with it is enhanced, that his/her self esteem is increased and/or that negative thoughts and/or negative tension, sadness, unhappiness/discontent and irritability, and dysphoria are/is reduced. It also means that emotions are balanced and/or that the general, especially the mental, well-being and vitality is improved or maintained, as well as that the risk of mood swings is lessened, that the positive mood is retained, and that one feels energetic and motivated.

"Tension reducer, sadness reducer, unhappiness/discontent reducer, irritability reducer, dysphoria reducer" means that (chronic) tension and worrying are reduced or alleviated. Hypervigilance syndrome, including restlessness and muscle tension, are also reduced or relieved. Social and other phobias are also at least partially resolved. In general, the social environment is experienced as less threatening. The person is emotionally relaxed, experiences comfort and enjoys company and contact with other people. In general, the person feels energetic and motivated to conduct daily tasks.

A "relaxant" works by completely or partially correcting a person's circadian rhythm (biorhythm) which has been disturbed due to jet-lag or shift work. A relaxant will at least partially prevent or abolish the symptoms associated with such disturbances, i.e. impairment of cognitive function and memory, mental and physical fatigue, and improve overall quality of life and vital energy. Thus, the lipophilic *Nigella sp.* preferably *Nigella sativa* seed extracts may also be used to prevent and/or abolish impairment of cognitive function and memory, to prevent and/or abolish mental and physical fatigue, and to improve overall quality of life and vital energy.

A further embodiment of the present invention relates to the use of lipophilic *Nigella sp.,* preferably *Nigella sativa* seed extracts and to the use of compositions containing them as "condition improvers", i.e. as means to reduce irritability and tiredness, to reduce, prevent or alleviate physical and mental fatigue, to favour undisturbed sleep, that is to act against insomnia and sleep disorders and to improve sleep, and to increase energy in more general terms, especially to increase brain energy production, in diseased or normal healthy individuals. Moreover, such "condition improvers" are to be used for cognition improvement in general, and especially for maintenance or improvement of attention and concentration, of memory and of the capacity for remembering, of learning ability, of language processing, of problem solving and of intellectual functioning; for improvement of short-term memory; for increasing mental alertness; for increasing the ability to focus and mental sharpness, for enhancing mental vigilance; for reducing mental fatigue; for supporting cognitive wellness, for maintaining balanced cognitive function, for the regulation of hunger and satiety as well as for the regulation of motor activity.

Thus, a preferred aspect the invention relates to the use of lipophilic *Nigella sativa* seed extracts as mood balancing agents and/or stress relievers.

In a further preferred embodiment of the present invention the lipophilic *Nigella sativa* seed extracts are used for maintaining circadian rhythms in humans, for alleviating and/or for preventing the symptoms associated with a disturbed circadian rhythm in humans. Thus, mood is stabilized and an emotional balance is achieved to cope with daily life stress and to maintain physical and psychological performance. Furthermore, the symptoms associated with a disturbed circadian rhythm, such as impairment of cognitive function and memory, and mental and physical fatigue, are alleviated and/or prevented so that the overall quality of life is improved. These persons also benefit from maintaining vital energy. Also, deregulation of circadian rhythms induced by long-distance flights (jet-lag) as well as by shift-work and the symptoms associated with it are alleviated and/or prevented.

Another preferred aspect of the invention relates to the use of the lipophilic *Nigella sativa* seed extracts for the manufacture of a composition, for use as an antidepressant to alleviate mood status and/or maintain a healthy mood.

In another embodiment, an effective dose of lipophilic *Nigella sp.,* preferably *Nigella sativa* seed extracts may especially be used for maintaining mental well-being, for maintaining a balanced cognitive function, for helping to retain a positive mood, relaxation and for supporting cognitive wellness.

The extracts of this invention improve, enhance and support physiological neurotransmission, especially in the central nervous system, and therefore may alleviate mental malfunction.

### VETERINARY USES

Another aspect of this invention are veterinary uses of the lipophilic *Nigella sp.,* preferably *Nigella sativa* seed extract. Animals may exhibit adverse behavioral and or physiological reactions to stressful situations. For example, animals raised in mass production environments, or being transported, can have a decline in meat or milk quantity or quality. Stressed poultry can resort to feather picking, reduced egg laying and cannibalism. Many animals can become aggressive or display obsessive-compulsive behaviors. The extracts of this invention, by acting on neurotransmitters, can relieve these unwanted behaviours and physiologies in animals.

In a preferred embodiment of the present invention the lipophilic *Nigella sativa* seed extracts are administered for preventing stress in farm animals, in mass production livestock husbandry, during transport to slaughter and/or for preventing quality loss of meat of said farm animals during transport to slaughter.

In another preferred embodiment of the present invention the lipophilic *Nigella sativa* seed extracts are administered to pets or companion animals for reduction of stress, tension and aggressiveness and compulsive behavior exhibited under stressful conditions, such as separation, change or loss of the owner, during holiday separation and husbandry in so called "animal hotels", husbandry in animal shelters or refuges.

Pet animals and farm animals can be in conditions which particularly benefit from enhanced or improved neurotransmission. Such conditions e.g. occur after capture or transport or with housing, when the animals develop analogous disorders and are distressed or aggressive, or display stereotypic behaviours, or anxiety, tension, sadness, unhappiness/discontent and irritability, dysphoria and obsessive-compulsive behaviour.

Thus, the lipophilic *Nigella sativa* seed extracts can be used in general as antidepressants for animals including humans, preferably for humans and other mammals, particularly pet animals and farm animals.

Another embodiment of this invention is method for preventing stress in farm animals in mass production livestock husbandry, during transport to slaughter and/or for preventing quality loss of meat of said farm animals during transport to slaughter, comprising administering an effective dose of a lipophilic *Nigella sativa* seed extract to farm animals which are in need thereof. The farm animals are preferably poultry, cattle, sheep, goats and swine.

In another preferred embodiment of the present invention the lipophilic *Nigella sativa* seed extract is administered to poultry for preventing feather picking and cannibalism resulting e.g. in losses of meat quality and egg production. Thus, another aspect of this invention is a method for preventing loss of egg production, feather picking and cannibalism and losses of meat quality upon transport stress amongst poultry, comprising administering an effective dose of a lipophilic *Nigella sativa* seed extract to poultry which is in need thereof.

Another aspect of this invention is a method for preventing and/or alleviating stress in aquaculture comprising the step of administering an effective dose of a lipophilic *Nigella sativa* seed extract to animals which are in need thereof, wherein the animals are fish or shrimp.

Yet another aspect of this invention is a method for reducing stress, tension, aggressiveness and/or compulsive behavior in pet animals under stressful conditions, such as separation, change or loss of the owner, during holiday separation and husbandry in so-called "animal hotels", husbandry in animal shelter stations and other conditions of dense husbandry and breeding, comprising the step of administering an effective dose of a lipophilic *Nigella sativa* seed extract to pet animals which are in need thereof, especially to cats and dogs which are in need thereof.

Still another aspect of this invention is a method for preventing and/or reducing symptoms associated with stressful conditions in animals used for the fur industry, preferably for minks, foxes and/or hares by administering a lipophilic *Nigella sativa* seed extract.

For animals, the lipophilic *Nigella sativa* seed extracts are in preferably administered as fortified feed or fortified beverages (e.g. as addition to the drinking water).

### Nigella sativa extracts

Lipophilic *Nigella sativa* seed extracts may be obtained in accordance with methods well-known in the art, e.g., by (an) extraction with solvents like methanol, ethanol, ethyl acetate, diethylether, n-hexane, methylenechloride, or with supercritical fluids like carbon dioxide (pure or in mixture with other solvents such as alcohols) or dinitrogen oxide, (b) hydrodistillation for obtaining essential oils or (c) extraction/distillation with hot gases like nitrogen.

The lipophilic *Nigella sativa* seed extracts can be of natural or synthetic or mixed (viz. partly natural, partly synthetic) origin, i.e., they can, apart from being obtained by extraction of plants and fractionation, be chemically synthesized and, if desired, mixed together in any desired quantities. They can be prepared and used in any desired purities and concentrations, e.g. as solutions containing them in concentrations as low as, e.g., 10% (w/w) or less, or up to nearly 100% (w/w).

Preferred are *Nigella sativa* extracts containing a high proportion of at least one of their volatile components. More preferred are *Nigella sativa* extracts containing at least a total of 70 weight-% of volatile components based on the total weight of the extract.

The composition of the present invention is preferably in the form of nutritional composition, such as fortified food, fortified feed, or fortified beverages, or in form of fortified liquid food/feed for animals including humans.

The dietary and pharmaceutical compositions according to the present invention may be in any solid, semisolid or liquid galenic form that is suitable for administering to the animal body including the human body, especially in any form that is conventional for oral administration, e.g. in solid form, such as (additives/supplements for) food or feed, food or feed premix, fortified food or feed, tablets, pills, granules, dragées, capsules, and effervescent formulations such as powders and tablets, or in liquid form such as solutions, emulsions or suspensions as e.g. beverages, pastes and oily suspensions. The pastes may be encapsulated in hard or soft shell capsules, whereby the capsules feature e.g. a matrix of (fish, swine, poultry, cow) gelatin, polysaccharides like starches or pullulan, or cellulose derivatives like hydroxypropyl-methyl-cellulose. Examples for other application forms are forms for transdermal, parenteral or injectable administration. The dietary and pharmaceutical compositions may be in the form of controlled (delayed) release formulations.

The dietary compositions according to the present invention may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellyfying agents, gel forming agents, antioxidants and antimicrobials.

Examples of fortified foods are vegetable juices, spicy cereals, and bakery items, such as spicy cookies or breads. Examples of dietary supplements are tablets, pills, granules, dragées, effervescent formulations and in specific capsules, in the form of non-alcoholic drinks, such as vegetable juices or other drinks or teas, in the form of liquid food, such as soups.

Beverages encompass non-alcoholic and alcoholic drinks as well as liquid preparations to be added to drinking water and liquid food. Non-alcoholic drinks are e.g. soft drinks, sport drinks, vegetable juices (e.g. tomato juice), and teas. Liquid foods are e.g. soups.

In addition to *Nigella sativa* extract, the compositions according to the present invention may further contain conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like. The carrier material can be organic or inorganic inert carrier material suitable for oral/parenteral/injectable administration.

For humans a suitable daily dosage of lipophilic *Nigella sativa* seed extracts or their volatile components for the purposes of the present invention may be within the range from 0.001 mg per kg body weight to about 100 mg per kg body weight per day. More preferred is a daily dosage of about 0.01 to about 50 mg per kg body weight, and especially preferred is a daily dosage of about 0.05 to 10.0 mg per kg body weight.

In solid dosage unit preparations for humans, the lipophilic *Nigella sativa* seed extract is suitably present in an amount from about 0.1 mg to about 6000 mg, preferably from about 1 mg to about 1000 mg, most preferably from about 25 mg to 500 mg per dosage unit. For relief of symptoms associated with conditions as mentioned herein, the lipophilic *Nigella sativa* seed extract is taken once or twice per day together with a meal for at least one week and up to 6-12 months. For prevention of occurrence of symptoms associated with conditions as mentioned herein and for the maintenance of a generally relaxed state, consumption on a regular basis is suitable.

In dietary compositions, especially in food and beverages for humans, the lipophilic *Nigella sativa* seed extract is suitably present in an amount of from about 0.0001 (1 mg/kg) to about 5 weight-% (50 g/kg), preferably from about 0.001 % (10 mg/kg) to about 1 weight-%, (10 g/kg) more preferably from about 0.01 (100 mg/kg) to about 0.5 weight-% (5 g/kg), based upon the total weight of the food or beverage. For relief of symptoms associated with conditions as defined above, the food product is taken once or twice per day at least for one to three weeks or on a regular basis, i.e. at least once daily.

In food and drinks in a preferred embodiment of the invention the amount of the lipophilic *Nigella sativa* seed extracts is 10 to 30 mg per serving, i.e. 120 mg per kg food or drink. The food product is taken once or twice per day at least for one to three weeks or preferably on a regular basis of at least once daily.

For animals excluding humans, a suitable daily dosage of a lipophilic *Nigella sativa* seed extract may be within the range from 0.001 mg per kg body weight to about 1000 mg per kg body weight per day. More preferred is a daily dosage of about 0.1 mg to about 500 mg per kg body weight, and especially preferred is a daily dosage of about 1 mg to 100 mg per kg body weight. To prevent and reduce symptoms associated with stressful conditions, such as mass production livestock husbandry and fur industry husbandry or aquaculture, the product containing the lipophilic *Nigella sativa* seed extract is given over the animal's entire lifetime until slaughter. Especially in the case, where farm animals, such as poultry, cattle, sheep, goats and swine, especially cattle and swine, are transported to slaughter, they should be administered a daily dosage of about 3 to 800 mg/kg body weight of the extract, preferably during transportation, more preferably at least 3 days before transportation and during transportation.

For pets, under stressful conditions as in animal shelter farms or pet shops, the product should be given for at least 1-3 weeks, or over the whole husbandry period. Under conditions of short-term stress, such as holiday separation, husbandry in animal "holiday hotels", visits to or stays in veterinarian clinics, the product may be given at least 3 days, and preferably 7 days, before the stressful event.

The invention is illustrated further by the following non-limiting examples.

### Examples

### Example 1:

### Preparation of Nigella sativa extracts

Lipophilic *Nigella sativa* seed extracts can be prepared from the seeds by hydrodistillation (steam distillation), extraction with organic solvents (such as but not limited to ethanol, ethyl acetate, or SF-CO₂) according to commonly available literature.

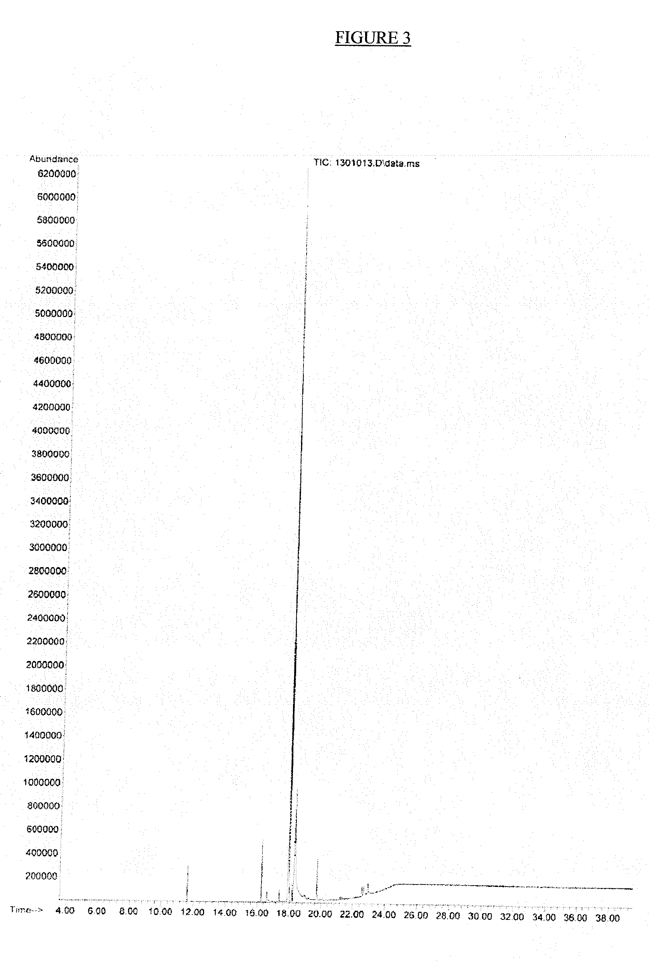

A GCMS chromatogram of a commercially available *Nigella sativa* extract from Analytikon containing fatty acids and 0.01 % (w/w) thymoquinone is shown in FIGURE 3.

### Extract I

10g of *Nigella sativa* seeds were extracted with (9/1) resulting in 2.2g of a lipophilic extract.

### Example 2

### Serotonin uptake inhibition by Nigella sativa extracts

HEK-293 cells stably expressing the human serotonin re-uptake transporter (hSERT) were obtained from R. Blakely, Vanderbilt University, USA. The cells were routinely grown in Dulbecco's Modified Eagle's Medium, purchased from Bioconcept, Allschwil, Switzerland containing 10% fetal calf serum, penicillin, streptomycin, L-glutamine and the antibiotic G418 and passaged by trypsinisation. 1 day prior to the assay cells were seeded in the above mentioned medium. Immediately prior to the assay the medium was replaced by Krebs-Ringer bicarbonate buffer, purchased from Sigma Chemicals Ltd., supplemented with 35 µM pargyline, 2.2 mM CaCl₂, 1 mM ascorbic acid and 5 mM N-2-hydroxyethylpiperazine-N'-2-ethanesulfonic acid ("Hepes" buffer). Serotonin uptake into the cells was determined by addition of radio-labeled (³H) serotonin (Amersham Biosciences GE Healthcare, Slough, UK) to a concentration of 20 nM, and incubation for 30 minutes at room temperature. Following removal of unincorporated label by gentle washing three times with the above buffer, incorporated serotonin was quantified by liquid scintillation counting.

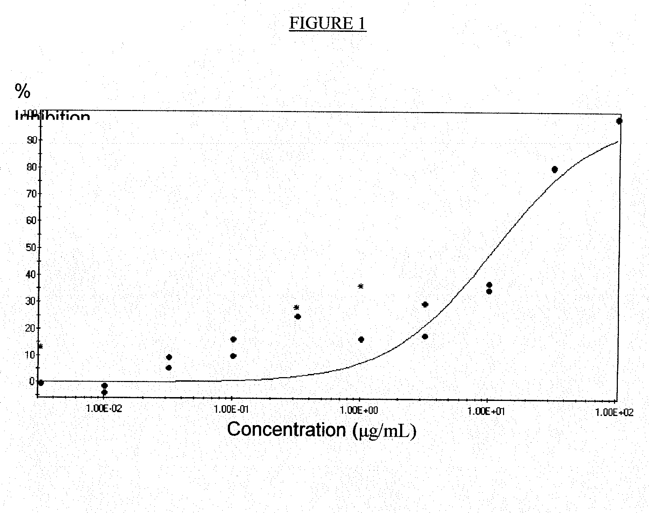

Serotonin uptake via the transporter was inhibited by the *Nigella sativa* extract I (from Example 1) in a dose dependent manner. The measured IC₅₀ values for inhibition of serotonin uptake by *Nigella sativa* extract is 14.14 ± 4.5 µg/mL and is shown in FIGURE 1. The data show, that an lipophilic *Nigella sativa* seed extract may have anti-depressant and mood lifting effects.

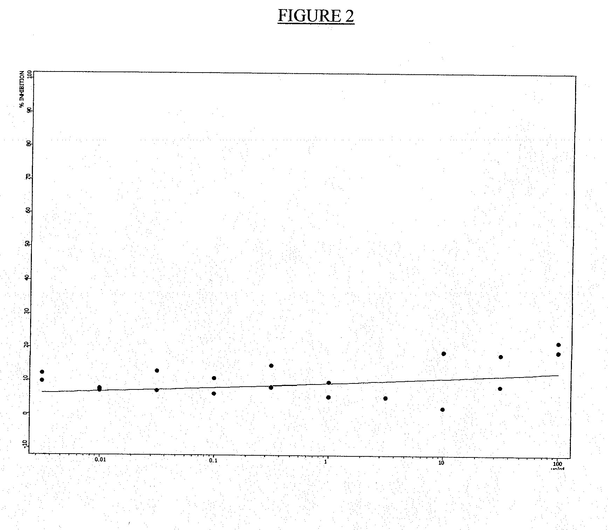

In contrast, a cold pressed oil of the seeds of black cumin (Abstswinder Schwarzkümmelöl, Germany) containing mostly polyunsaturated fatty acids and 0.3% (w/w) thymoquinone did not have this effect yielding an IC50 of 129.4 µg/mL. (See FIGURE 2)

### Example 3:

### Preparation of a soft gelatin capsule

A soft gelatin capsule may be prepared comprising the following ingredients:

| **Ingredient** | **Amount per Capsule** |
|---|---|
| *Nigella sativa* extract | 150 mg |
| Lecithin | 50 mg |
| Soy bean oil | 250 mg |

Two capsules per day for 3 months may be administered to a human adult for the treatment of mild chronic dysthymia.

### Example 4

### Preparation of a soft gelatin capsule

A soft gelatin capsule may be prepared comprising the following ingredients:

| **Ingredient** | **Amount per Capsule** |
|---|---|
| *Nigella sativa* extract | 200 mg |
| Evening primrose oil | 300 mg |
| Vitamin B₆ | 100 mg |

One capsule per day, preferably during the second half of the menstrual cycle, may be taken for 14 days for the treatment of premenstrual syndrome and premenstrual dysphoric disorder.

### Example 5

### Preparation of an enriched tomato juice

| **Ingredient** | **Amount [g]** |
|---|---|
| *Nigella sativa* Extract | 0.12 |
| **Tomatoe juice ad** | **1000** |

The juice contains ca. 30 mg *Nigella sativa* extract per serving (250 ml).

As a strengthener and for general well-being 2 servings per day (240 ml) is recommended.

### Example 6:

### Dry dog food

Commercial basal diet for dogs (e.g. Mera Dog "Brocken", MERA-Tiernahrung GmbH, Marienstraße 80-84, D-47625 Kevelaer-Wetten, Germany) is sprayed with a solution of *Nigella sativa* extract in an amount sufficient to administer to a subject a daily dose of 50 mg per kg body weight, based on the weight of the *Nigella sativa* extract or its volatile components concentrate. The food composition is dried to contain dry matter of about 90 % by weight. For an average dog of 10 kg body weight to consume approx. 200g dry feed per day, the dog food contains approx. 2500 mg *Nigella sativa* extract or its volatile components/kg food. For heavier dogs, the feed mix is prepared accordingly.

### Example 7

### Wet cat food

Commercial basal diet for cats (e.g. Happy Cat "Adult", Tierfeinnahrung, Südliche Hauptstraße 38, D-86517 Wehringen, Germany) is mixed with a solution of *Nigella sativa* extract or its volatile components in an amount sufficient to administer to a subject a daily dose of 100 mg per kg body weight, based on the weight of the dried *Nigella sativa* extract or its volatile components concentrate. For an average cat of 5 kg of body weight to consume approx. 400 g of wet food, the cat food contains 1250 mg/kg *Nigella sativa* extract. The food composition is dried to contain dry matter of about 90 % by weight.

### Example 8

### Dog treats containing Nigella sativa extract

Commercial dog treats (e.g. Mera Dog "Biscuit" for dogs as supplied by Mera Tiernahrung GmbH, Marienstrasse 80-84, 47625 Kevelaer-Wetten, Germany) are sprayed with a solution of *Nigella sativa* extract or its volatile components in an amount sufficient to administer to the treats 5 - 50 mg per g treats, based on the weight of the dried *Nigella sativa* extract or its volatile components concentrate. The food composition is dried to contain dry matter of about 90% by weight.

### Example 9

### Cat treats containing Nigella sativa extract

Commercial cat treats (e.g. Whiskas Dentabits for cats as supplied by Whiskas, Masterfoods GmbH, Eitzer Str. 215, 27283 Verden/Aller, Germany) are sprayed with a solution of *Nigella sativa* extract or its volatile components in an amount sufficient to administer to the treats 5 - 50 mg per g treats, based on the weight of the dried *Nigella sativa* extract or its volatile components concentrate. The food composition is dried to contain dry matter of about 90% by weight.

### Example 10

### Preparation of a broccoli soup

| **Ingredient** | **Amount** |
|---|---|
| Broccoli | 300 g |
| Onion powder | 14 g |
| Garlic powder | 5 g |
| Vegetable stock | 850 g |
| Potato flakes | 50 g |
| Powdered almonds | 20 g |
| Salt | 10 g |
| Citric acid | 0.5 g |
| Nigella *sativa* extract | 0.5 g |
| In total | 1250 g |

### Preparation:

The vegetable is cooked for 15-20 min, mixed and purreed with the vegetable stock. Then, the other ingredients are added and heated again, before the soup is filled into tins.

1 serving (250 g) contains 100 mg of *Nigella sativa* Extract.

### Example 11:

### Cracker with Pepper flavor

| | **Ingredients** | **Quantity [g]** |
|---|---|---|
| **1.0** | **Dough** | |
| 1.1 | Wheat flour type 550 | 540.0 |
| 1.2 | Skim milk powder | 30.0 |
| 1.3 | Icing sugar | 13.0 |
| 1.4 | Whey powder sweet | 12.0 |
| 1.5 | Yeast powder autolysed | 10.0 |
| 1.6 | Salt | 4.6 |
| 1.7 | Backing powder | 4.0 |
| 1.8 | Nigella *sativa* Extract | 0.4 |
| 1.9 | Malt Powder | 7.2 |
| 1.10 | Mixed Spices * | 10.0 |
| **2.0** | **Water (RT)** | 100.0 |
| **3.0** | **Fat/Flavor Mixture** | |
| 3.1 | Vegetable Fat (biscofin) | 94.0 |
| 3.2 | Flavor -PAPRIKA- 530214 E Guivaudan) | 1.5 |
| **4.0** | **Syrup Solution** | |
| 4.1 | Water | 56.8 |
| 4.2 | Glucose Syrup DE 38 | 14.4 |
| 4.3 | Lactic Acid | 1.6 |
| **5.0** | **Ammonium carbonate solution** | |
| 5.1 | Water | 88.5 |
| 5.2 | Ammonium carbonate | 5.4 |
| **6.0** | **Dusting Powder** | |
| 6.1 | Wheat flour Type 550 | 82.0 |
| 6.2 | Salt fine ground | 0.6 |
| 6.3 | Vegetable fat | 17.4 |
| | | |
| | Total (dough) | 1093.4 |

### Mixed Spices

| | | |
|---|---|---|
| ***** | **Ingredients** | **Quantity [g]** |
| **8.0** | **Spice Mixture** | |
| 8.1 | Onion powder | 2.50 |
| 8.2 | Garlic powder | 3.10 |
| 8.3 | White pepper | 0.775 |
| 8.4 | Oregano | 0.625 |
| 8.5 | Parsley | 0.625 |
| 8.6 | Nutmeg | 2.375 |
| | **Total** | **10.00** |

### Preparation of dry mixture and solutions

- Sieve all dry ingredients and add 1.1 - 1.10 into Kenwood type mixer
- Mix the ingredients from position 3.0
- Heat the water (4.0) and dissolve the glucose syrup, add lactic acid
- Dissolve ammonium carbonate in water (5.1 - 5.2)

### Preparation of the dough

- Start the mixer before adding the water (2.0)
- Add the other ingredients (3.0, 4.0, 5.0) bit by bit according to the ingredient list
- Knead the dough for at least 5 min

### Form the dough

- Roll the dough in several layers (about 3) and sprinkle with dusting powder (6.0) between the layers
- Roll to a final thickness of 1.5 mm
- Prick holes into the dough and cut into pieces (ca. 2,5 cm x 2,5 cm) of one serving size

### Backing and drying

- Bake the crackers at 220 C° for 5-7 min
- Dry the crackers subsequent for 1,5 hours at 80 °; cool at RT

100 g of the ready-to-eat crackers contain 47 mg Nigella *sativa* extract.

## Claims

1. A dietary or pharmaceutical composition comprising a lipophilic *Nigella sp.* seed extract in a effective amount for the treatment of disorders connected to impaired or reduced neurotransmission in an animal.

2. The composition of claim 1 wherein the *Nigella sp.* seed extract is obtained by an extraction method selected from the group consisting of: liquid carbon dioxide under supercritical conditions, steam distillation/hydrodistillation yielding essential oils, ethanol extraction, ethyl acetate extraction, methyl-tert.-butylether, methanol, propane, butane, acetone and nitro oxide.

3. A dietary composition comprising a composition according to Claim 1 or 2, selected from the group consisting of: fortified food, spicy cereal bars, bakery items, cookies, dietary supplements, non-alcoholic drinks, soft drinks, sport drinks, vegetable juices, teas, liquid food, and soups.

4. A composition according to any of Claims 1-3 which is from *Nigella sativa.*

5. Use of a lipophilic *Nigella sp.* seed extract according to Claim 1 or 2 for the manufacture of a composition for the treatment of a disorder connected to impaired neurotransmission.

6. Use according to Claim 5, wherein the composition is an antidepressant, mood/vitality improver, a stress reliever, a condition improver, a reducer of anxiety, a reducer of tension, a reducer of sadness, a reducer of unhappiness/discontentedness, a reducer of irritability, a reducer of dysphoria, a reducer of obsessive-compulsive behaviour, a relaxant, a sleep improver and/or an insomnia alleviator.

7. A use according to Claim 5 or 6 wherein the composition prevents or normalizes circadian rhythm disruptions.

8. A use according to any of Claims 5-7 which is a veternary use.

9. A use according to any of Claims 5-8 wherein the *Nigells sp.* is *Nigella sativa.*

10. A method of treating a condition resulting from impaired neurotransmission comprising administering to an animal, including a human, an effective amount of a lipophilic *Nigella sativa* seed extract or of at least one of its volatile components.

11. The method of Claim 10 wherein the animal treated is selected from the group consisting of: farm animals, companion animals, aquaculture animals and animals used in the fur industry.

12. A method according to Claim 10 or 11 wherein the condition is selected from the group consisting of: depression, mood/vitality imbalance, a stress, anxiety, tension, sadness, unhappiness/discontentedness, irritability, dysphoria, dysthymia, obsessive-compulsive behaviour, sleep condition, improver, and insomnia.
